# EUROPEAN PATENT SPECIFICATION

(11) **EP 2 792 752 B1**
(45) Date of publication and mention of the grant of the patent: **04.04.2018**
(21) Application number: 12857270.8
(22) Date of filing: 11.12.2012
(51) Int. Cl.: C12Q 1/68

(54) **METHOD FOR THE DIAGNOSIS OF ACUTE RENAL DAMAGE**
VERFAHREN ZUR DIAGNOSE AKUTER NIERENSCHÄDEN
MÉTHODE POUR LE DIAGNOSTIC D'UNE LÉSION RÉNALE AIGUË

(30) Priority: 15.12.2011 ES 201132023
(43) Date of publication of application: 22.10.2014
(73) Proprietor: Fundación Para La Investigación Biomédica Del Hospital Universitario Ramón Y Cajal, 28034 Madrid (ES)
(72) Inventor: AGUADO FRAILE, Elia, 28034 Madrid (ES); RAMOS MUÑOZ, Miren Edurne, 28034 Madrid (ES); CANDELA TOHA, Angel Manuel, 28034 Madrid (ES); LIAÑO GARCIA, Fernando, 28034 Madrid (ES); GARCIA BERMEJO, María Laura, 28034 Madrid (ES)
(74) Representative: Hoffmann Eitle
(86) International application number: PCT/ES2012/070858
(87) International publication number: WO 2013/087961

(56) References cited:
- WO-A2-2011/027019
- WO-A2-2011/133036
- LAURA DENBY ET AL: "miR-21 and miR-214 Are Consistently Modulated during Renal Injury in Rodent Models", THE AMERICAN JOURNAL OF PATHOLOGY, vol. 179, no. 2, 1 August 2011 (2011-08-01), pages 661-672, XP055080332, ISSN: 0002-9440, DOI: 10.1016/j.ajpath.2011.04.021
- YU Q. CHEN ET AL: "Abated microRNA-195 expression protected mesangial cells from apoptosis in early diabetic renal injury in mice", JOURNAL OF NEPHROLOGY, vol. 25, no. 4, 4 October 2011 (2011-10-04), pages 566-576, XP055156522, ISSN: 1121-8428, DOI: 10.5301/jn.5000034
- J. M. LORENZEN ET AL: "Urinary miR-210 as a Mediator of Acute T-Cell Mediated Rejection in Renal Allograft Recipients", AMERICAN JOURNAL OF TRANSPLANTATION, vol. 11, no. 10, 3 October 2011 (2011-10-03), pages 2221-2227, XP055134470, ISSN: 1600-6135, DOI: 10.1111/j.1600-6143.2011.03679.x
- J. G. GODWIN ET AL: "Identification of a microRNA signature of renal ischemia reperfusion injury", PROCEEDINGS OF THE NATIONAL ACADEMY OF SCIENCES, vol. 107, no. 32, 22 July 2010 (2010-07-22), pages 14339-14344, XP055156529, ISSN: 0027-8424, DOI: 10.1073/pnas.0912701107
- SUI ET AL: "Microarray analysis of MicroRNA expression in acute rejection after renal transplantation", TRANSPLANT IMMUNOLOGY, ELSEVIER, NL, vol. 19, no. 1, 5 February 2008 (2008-02-05), pages 81-85, XP022533984, ISSN: 0966-3274, DOI: 10.1016/J.TRIM.2008.01.007
- ANONYMOUS: "Taqman array human MicroRNA Cards", INTERNET CITATION, 2010, pages 1-2, XP002672200, Retrieved from the Internet: URL:http://www3.appliedbiosystems.com/cms/ groups/mcb_marketing/documents/generaldocu ments/cms_054742.pdf [retrieved on 2012-03-23]
- "Agilent Human, Mouse, and Rat miRNA Microarrays", , 16 January 2009 (2009-01-16), pages 1-4, XP055005699, Retrieved from the Internet: URL:http://www.chem.agilent.com/Library/br ochures/5989-7688en_lo.pdf [retrieved on 2011-08-26]
- CHEN, Y. Q. ET AL.: 'Abated microRNA-195 expression protected mesangial cells from apoptosis in early diabetic renal injury in mice.' JOURNAL OF NEPHROLOGY. vol. 25, no. 4, July 2012, pages 566 - 576., XP055156522
- DENBY, L. ET AL.: 'miR-21 and miR-214 are consistently modulated during renal injury in rodent models.' AMERICAN JOURNAL OF PATHOLOGY. vol. 179, no. 2, August 2011, pages 661 - 672., XP055080332
- LONG, J. ET AL.: 'MicroRNA-29c is a signature microRNA under high glucose conditions that targets Sprouty Homolog 1, and its in vivo knockdown prevents progression of diabetic nephropathy.' THE JOURNAL OF BIOLOGICAL CHEMISTRY vol. 286, no. 13, April 2011, pages 11837 - 11848., XP055156526
- DU, B. ET AL.: 'High glucose down-regulates miR-29a to increase collagen IV production in HK-2 cells.' FEBS LETTERS. vol. 584, no. 4, February 2010, pages 811 - 816., XP026921763
- GODWIN, J. G. ET AL.: 'Identification of a microRNA signature of renal ischemia reperfusion injury.' PROCEEDINGS OF THE NATIONAL ACADEMY OF SCIENCES OF THE UNITED STATES OF AMERICA. vol. 107, no. 32, August 2010, pages 14339 - 14344., XP055156529
- CHOW, T.-F. F. ET AL.: 'The miR-17-92 cluster is over expressed in and has an oncogenic effect on renal cell carcinoma.' JOURNAL OF UROLOGY. vol. 183, no. 2, February 2010, pages 743 - 751., XP026833124
- LONG, J. ET AL.: 'Identification of microRNA-93 as a novel regulator of vascular endothelial growth factor in hyperglycemic conditions.' THE JOURNAL OF BIOLOGICAL CHEMISTRY vol. 285, no. 30, July 2010, pages 23457 - 23465, XP055156531
- LORENZEN, J. M ET AL.: 'Urinary miR-210 as a mediator of acute T-cell mediated rejection in renal allograft recipients.' AMERICAN JOURNAL OF TRANSPLANTATION: OFFICIAL JOURNAL OF THE AMERICAN SOCIETY OF TRANSPLANTATION AND THE AMERICAN SOCIETY OF TRANSPLANT. vol. 11, no. 10, October 2011, pages 2221 - 2227., XP055134470

## Description

### Field of the invention

The present invention is encompassed in the general field of biomedicine and relates particularly to a method for the diagnosis and/or prognosis of acute renal damage.

### State of the art

miRNAs are small, endogenously encoded RNAs (22-25 nucleotides) capable of recognizing messenger RNAs and thus negatively regulating protein expression within RNA-induced silencing complexes (RISCs) due to partial or complete complementarity with their target mRNA. Most miRNAs are transcribed by RNA Pol II from individual genes or from polycistronic transcripts for several of them at one time. They are generated as longer pre-miRs which are processed in the nucleus by ribonuclease III, move out to the cytoplasm by means of Exportin-5 and Ran-GTP-dependent mechanisms and are finally processed by another ribonuclease III into their mature form in the cytoplasm.

Their function is essential in a wide range of processes including embryonic development, stress response or strict regulation of physiological processes and, therefore, homeostasis maintenance of the body.

Very recently it has been demonstrated that miRNAs are key regulators in rapid and precise cell response to any type of stimulus including the lack of nutrients or hypoxia (Ivan M, Harris AL, Martelli F, Kulshreshtha R. Hypoxia response and microRNAs: no longer two separate worlds. J Cell Mol Med.;12(5A):1426-31, 2008).

Patent application WO2011012074 describes a series of miRNAs including, among them, miR-29a, as plasma markers for liver cancer, and a method for the diagnosis and evaluation of liver cancer based on the detection of at least one of said miRNAs.

Patent application WO2009036236 relates to a series of miRNAs including, among them, miR-146a, as plasma markers for liver cancer, and a method for the diagnosis and/or evaluation of different oncologic pathologies based on the detection of at least one of the described microRNAs.

Akkina, S. et al., "MicroRNAs in renal function and disease", Translational Research, 2011 Apr, Vol. 157, No. 4, pg 236-240 and Li, J.Y. et al., "Review: The role of microRNAs in renal disease", NEPHROLOGY (CARLTON), Sep 2010, Vol. 15, No.6, p599-608, describe the involvement of miRNAs in renal physiology and pathology.

JUAN, D. et al., "Identification of a microRNA panel for clear-cell renal cancer", UROLOGY, 2010 Apr, Vol.75, No. 4, pages 835-41, describes a series of miRNAs as markers for renal cancer.

DENBY et al. (AJP 2011, 179(2):661-672) and YU et al. (J NEPHROL. 2011, 25(4):566-576) disclose that miR-26b expression levels are reduced in the kidney tissue of mouse models for progressive renal fibrosis and diabetic nephropathy.

WO2011/027019 discloses a method for the diagnosis of acute renal damage comprising determining the expression level of at least one micro-RNA in a sample. Acute renal failure (ARF) as a syndrome is characterized by a sharp decrease in glomerular filtrate within days or weeks, being clinically expressed with the inability to excrete nitrogenated waste products and regulate fluid and electrolyte homeostasis.

ARF is one of the most serious problems among renal diseases in the developed world as it entails a high mortality of about 50%. About 30% of all ARF episodes occur in patients admitted to the ICUs as a result of multiple organ failure. In this last context, mortality increases to 80%.

The development of ARF is also one of the most common complications after cardiac interventions amounting to 30,000 a year in Spain and more than 1% of said interventions are conducted in this hospital. Virtually, all the patients that undergo intervention develop certain degree of ARF. The long-term progression of the patients depends on the severity of this post-operation ARF, resulting in mortality close to 60% in those cases that require dialysis after the cardiac intervention (Candela-Toha A, Elias-Martin E, Abraira V et al. Predicting Acute Renal Failure after Cardiac Surgery: External Validation of Two New Clinical Scores. Clin J Am Soc Nephrol; 3:1260-1265. 2008). Both cardiac surgery and kidney transplant are two "quasi" experimental situations for studing ATN in humans since the moment and duration of the ischemic stimulus are known and can also be monitored. All these morbi-mortality statistics have not changed significantly over the last decades and up until now, there is no effective therapy to prevent and/or reduce ATN in all these situations. This is due, in a large extent, to the lack of more precise renal damage markers other than the determination of creatinine and urea in serum used up until now. These conventional markers do not directly reflect cell damage nor do they reflect the compartment of the kidney tissue (tubule or endothelium) in which said damage occurs, they are only parameters indicative of an impaired renal function resulting from damage (Vaidya VS, Waikar SS, Ferguson MA, et al., Urinary Biomarkers for Sensitive and Specific Detection of Acute Renal Injury in Humans. Clin Transl Sci. ;1(3):200-208, 2008). In fact, it is possible that patients with subclinical renal damage are not identified as such because there is no significant alteration in serum creatinine and urea levels. Therefore, over recent years various studies are developed in order to identify and validate new markers for ARF such as NGAL, IL18, KIM, Cystatin C, VEGF or CXCL10, which seem to work as good markers in child populations without extra significant pathologies but not in adult population (Vaidya VS, Waikar SS, Ferguson MA, et al., Urinary Biomarkers for Sensitive and Specific Detection of Acute Renal Injury in Humans. Clin Transl Sci. 1(3):200-208, 2008).

Everything described above justifies the need to identify and validate new, more precise biomarkers for renal damage progression that are indicative of the tissue compartment in which the damage occurs and the degree of the damage and/or recovery, the determination of which is also fast, simple and without a biopsy on the patient being needed.

### Description of the invention

The present invention relates to a method for the diagnosis of acute renal damage as a result of ischemic or toxic etiology which comprises determining the expression level of at least miR-26b in a serum or blood sample isolated from a subject and comparing said expression level with a control value, wherein the reduction of said expression level is indicative of acute renal damage as a result of ischemic or toxic etiology. In the present invention, acute renal damage is understood as any damage caused by a sharp decrease in kidney function within hours or days, as a decrease in glomerular filtrate or an accumulation of serum nitrogenated products, or as an inability to regulate homeostasis.

In a preferred embodiment of the present invention, the expression of the micro-RNA or micro-RNAs is determined by means of quantitative PCR.

In another preferred embodiment of the present invention, the expression level of the micro-RNA is determined by means of RNA microarrays.

In another preferred embodiment, the method of the present invention comprises determining the expression levels of miR-26b, miR-29a, miR-454, miR-146a, miR-27a, mi-R93 and miR-10a collectively. In another more preferred embodiment, the reduction in the expression level of at least one of the micro-RNAs is indicative of acute renal damage.

In another aspect, the present invention relates to the use of at least miR-26b for the diagnosis of acute renal damage as a result of ischemic or toxic etiology in a serum or blood sample isolated from a subject. In another aspect, the present invention relates to the use of miR-26b, miR-29a, miR-454, miR-146a, miR-27a, mi-R93 and miR-10a for the diagnosis of acute renal damage, collectively.

In another aspect, the present invention relates to the in vitro use of a kit for the diagnosis of acute renal damage as a result of ischemic or toxic etiology according to the method of claims 1-5, comprising the probes and primers required for determining the expression level of at least miR-26b.

### Description of the Drawings

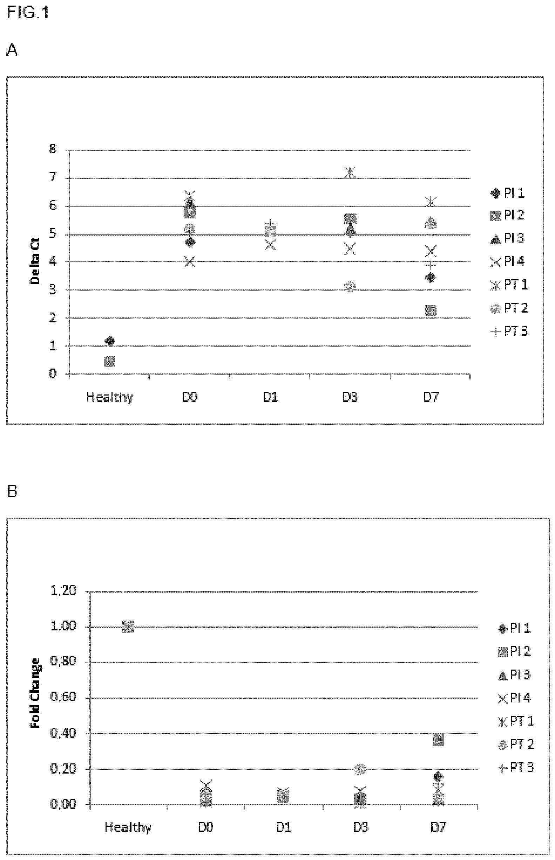

Figure 1 shows the serum miR-26b expression in ARF patients with ischemic etiology (PI) and toxic etiology (PT) from the time of diagnosis (DO) to 7 days after diagnosis, compared with the miRNA expression in two pools of healthy individuals (healthy). a) Data of amplification cycle in which miRNA expression is detected in all samples (crossing threshold,CT) with respect to a synthetic microRNA used as an internal control of the technique, b) folds of miRNA expression in ARF patients with respect to healthy individuals which are equalized to 1 by means of a exponential mathematical formula.

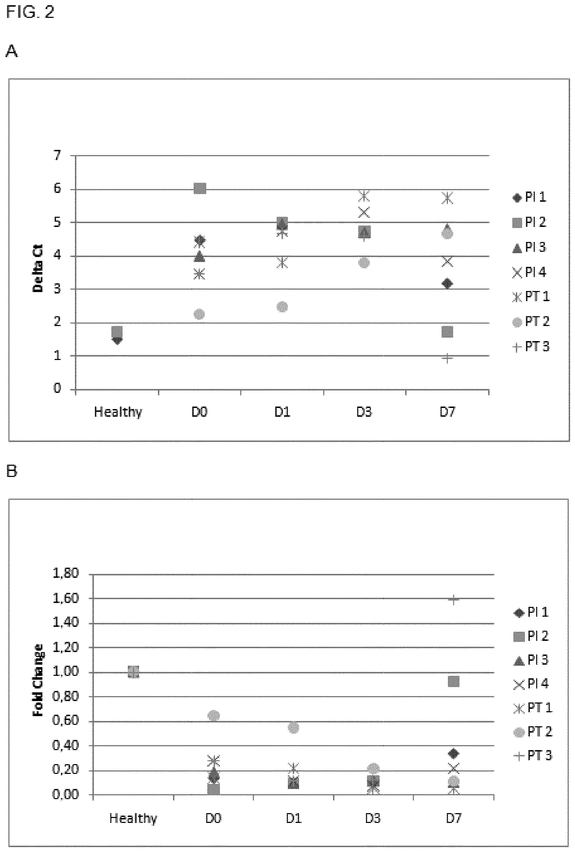

Figure 2 shows the serum miR-29a expression in ARF patients with ischemic etiology (PI) and toxic etiology (PT) from the time of diagnosis (DO) to 7 days after diagnosis, compared with the miRNA expression in two pools of healthy individuals (healthy). a) Data of the amplification cycle in which miRNA expression is detected in all samples (crossing threshold, CT), with respect to a synthetic microRNA used as an internal control of the technique, b) folds of miRNA expression in ARF patients with respect to healthy individuals which are equalized to 1 by means of a exponential mathematical formula.

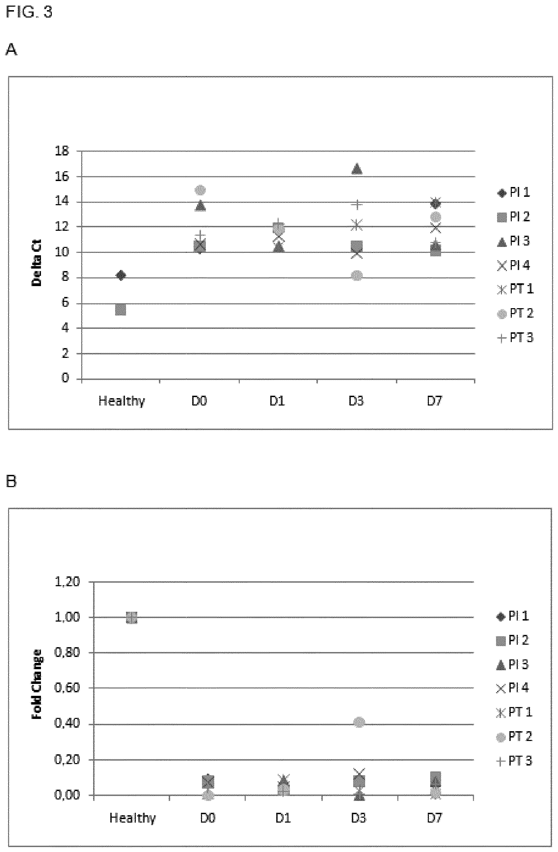

Figure 3 shows the serum miR-454 expression in ARF patients with ischemic etiology (PI) and toxic etiology (PT) from the time of diagnosis (DO) to 7 days after diagnosis, compared with the miRNA expression in two pools of healthy individuals (healthy). a) Data of the amplification cycle in which miRNA expression is detected in all samples (crossing threshold, CT), with respect to a synthetic microRNA used as an internal control of the technique, b) folds of miRNA expression in ARF patients with respect to healthy individuals which are equalized to 1 by means of a exponential mathematical formula.

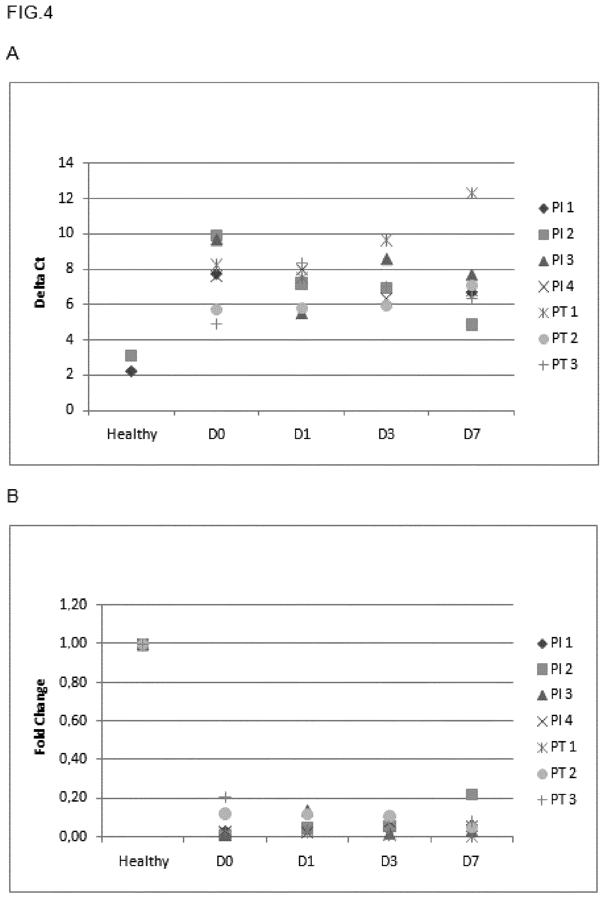

Figure 4 shows the serum miR-146 expression in ARF patients with ischemic etiology (PI) and toxic etiology (PT) from the time of diagnosis (DO) to 7 days after diagnosis, compared with the miRNA expression in two pools of healthy individuals (healthy). a) Data of the amplification cycle in which miRNA expression is detected in all samples (crossing threshold, CT), with respect to a synthetic microRNA used as an internal control of the technique, b) folds of miRNA expression in ARF patients with respect to healthy individuals which are equalized to 1 by means of a exponential mathematical formula.

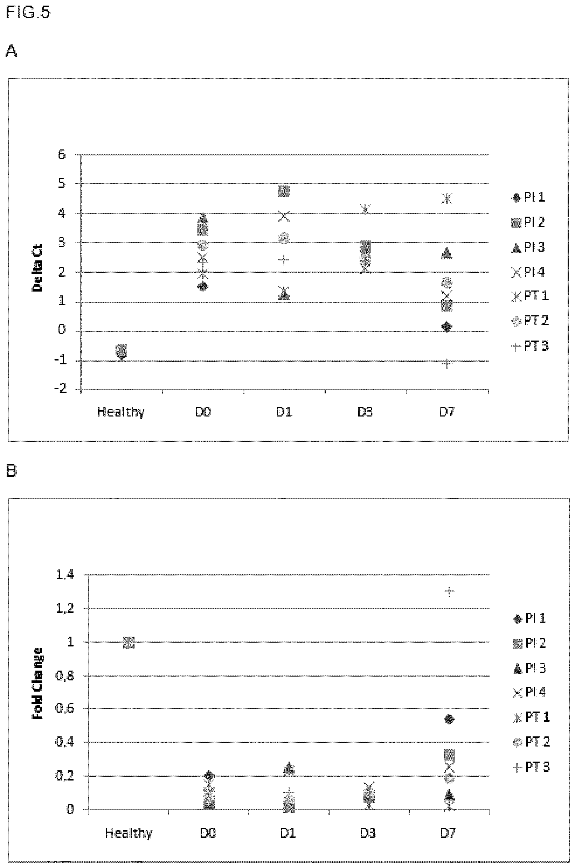

Figure 5 shows the serum miR-27a expression in ARF patients with ischemic etiology (PI) and toxic etiology (PT) from the time of diagnosis (DO) to 7 days after diagnosis, compared with the miRNA expression in two pools of healthy individuals (healthy). a) Data of the amplification cycle in which miRNA expression is detected in all samples (crossing threshold, CT), with respect to a synthetic microRNA used as an internal control of the technique, b) folds of miRNA expression in ARF patients with respect to healthy individuals which are equalized to 1 by means of a exponential mathematical formula.

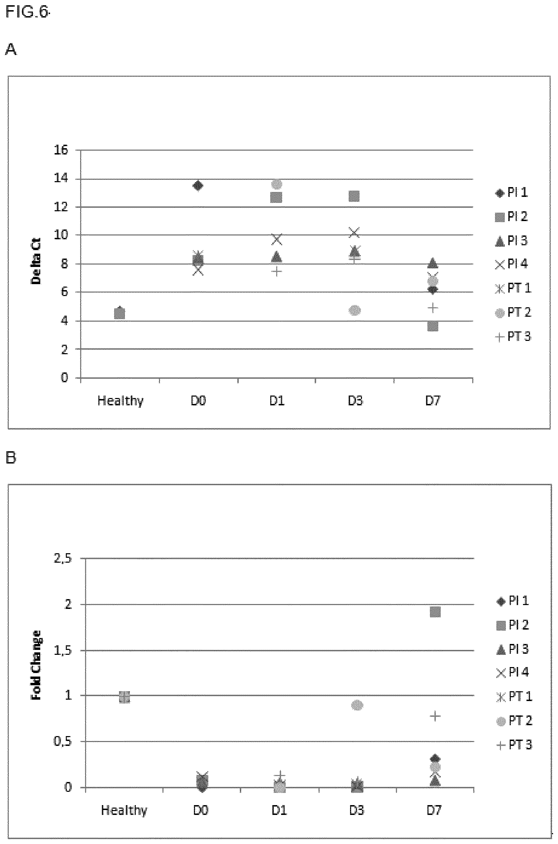

Figure 6 shows the serum miR-93 expression in ARF patients with ischemic etiology (PI) and toxic etiology (PT) from the time of diagnosis (DO) to 7 days after diagnosis, compared with the miRNA expression in two pools of healthy individuals (healthy). a) Data of the amplification cycle in which miRNA expression is detected in all samples (crossing threshold, CT), with respect to a synthetic microRNA used as an internal control of the technique, b) folds of miRNA expression in ARF patients with respect to healthy individuals which are equalized to 1 by means of a exponential mathematical formula.

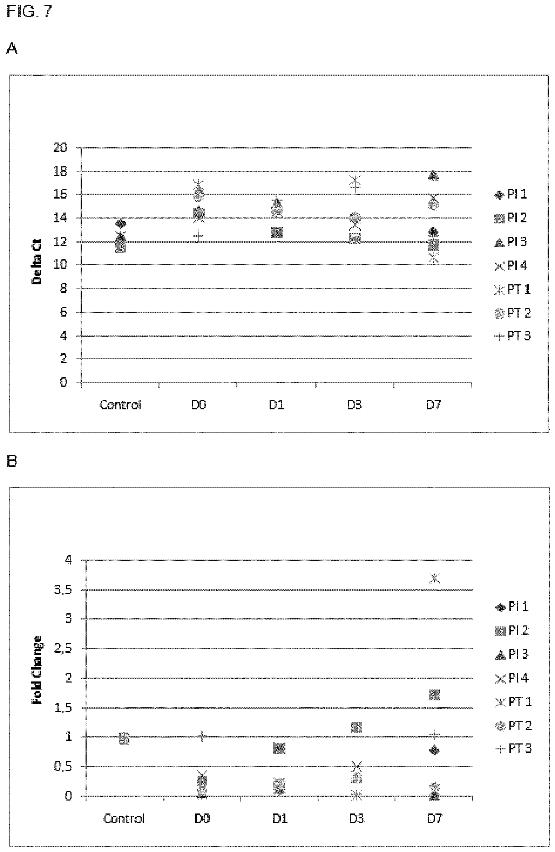

Figure 7 shows the serum miR-10a expression in ARF patients with ischemic etiology (PI) and toxic etiology (PT) from the time of diagnosis (DO) to 7 days after diagnosis, compared with the miRNA expression in two pools of healthy individuals (healthy). a) Data of the amplification cycle in which miRNA expression is detected in all samples (crossing threshold, CT), with respect to a synthetic microRNA used as an internal control of the technique, b) folds of miRNA expression in ARF patients with respect to healthy individuals which are equalized to 1 by means of a exponential mathematical formula.

### Detailed description of the invention

An experiment for large scale microRNA study was first conducted using RNA obtained from acute renal failure patients with different etiologies. The samples used for this method consisted of:
- Two samples from acute renal failure patients with ischemic etiology at the time of maximum renal damage indicated by means of common clinical diagnosis parameters, such as serum creatinine.
- Two samples from these same patients 7 or 10 days after renal failure, when the organ function recovered, again estimated by means of serum creatinine.
- Two samples from a patient with renal failure caused by nephrotoxic substances, one at the time of maximum damage and the other when renal function recovered.
- A sample from a patient having renal failure caused by sepsis, taken at the time of maximum organ damage.
- Two groups consisting of 5 healthy people that are used as control.

The experiment for large scale serum microRNA study was conducted by means of quantitative PCR using the Taqman Low Density Array (TLDAs) platforms of Applied Biosystems. By analyzing the data obtained, the microRNAs of interest were selected for study.

The expression data of the selected microRNAs obtained in the previous experiment was subsequently confirmed. To that end, samples from patients other than those mentioned above were used and they consist of:
- Four acute renal failure patients with ischemic etiology, referred to as PI1, PI2, PI3, PI4. From each patient, samples on day 0, the time of diagnosis, 1, 3, 5 and 7 days after the diagnosis of renal failure were used.
- Three patients having acute renal failure caused by nephrotoxic substances, referred to as PT1, PT2, PT3. From each patient, samples on day 0, the time of diagnosis, 1, 3, 5 and 7 days after the diagnosis of renal failure were used.
- Two control groups, each consisting of 10 healthy people.

To confirm the data, quantitative PCR was performed using individual probes for each microRNA of LNA technology (Exiqon).

As shown in Figures 1-4, serum miRNA expression decreases in ARF patients with respect to healthy controls.

Figure 5 shows that miR-27a expression decreases in ARF patients with respect to healthy controls. It is important to note that miR-27a expression within 7 days was more variable among patients and some of them had a tendency to recover expression values of healthy individuals.

miR-93 expression (Figure 6) decreased in ARF patients with respect to healthy controls, and it should be also noted that on day 7 this miRNA showed a tendency to recover values that are close to the controls in some patients.

miR-10a expression (Figure 7) decreased in some ARF patients with respect to healthy controls, this tendency was not observed in an ischemic patient and it is indeed important to note that on day 7 the miRNA expression levels recovered in several patients.

Furthermore, the area under the curve values were calculated by ROC curve analysis for miRNA expression in different patients.

Table 1 shows that these miRNAs had an ARF diagnosis value regardless of the etiology of the ARF, with specificity and sensitivity much greater than serum creatinine (marker used today).

**Table 1: ROC curve analysis for miRNAs in ARF patients in ICU on day 0**

| Contrast result variables | Area | Standard error ^{a} | Asymptotic Sig. ^{b} | 95% Asymptotic Confidence Interval | |
|---|---|---|---|---|---|
| | | | | Lower limit | Upper limit |
| miR_101_D0 | 1.000 | 0.000 | 0.000 | 1.000 | 1.000 |
| miR_210_D0 | 0.761 | 0.120 | 0.043 | 0.526 | 0.995 |
| miR_126_D0 | 0.900 | 0.061 | 0.002 | 0.780 | 1.020 |
| miR_26b_D0 | 1.000 | 0.000 | 0.000 | 1.000 | 1.000 |
| miR_29a_D0 | 0.757 | 0.095 | 0.046 | 0.572 | 0.943 |
| miR_146a_D0 | 1.000 | 0.000 | 0.000 | 1.000 | 1.000 |
| miR_27a_D0 | 0.786 | 0.091 | 0.027 | 0.608 | 0.964 |
| miR_93_D0 | 0.771 | 0.099 | 0.036 | 0.577 | 0.965 |
| miR_10a_D0 | 1.000 | 0.000 | 0.000 | 1.000 | 1.000 |
| miR_127_D0 | 0.714 | 0.122 | 0.097 | 0.474 | 0.954 |

Data of Table 2 demonstrates that these miRNAs were more sensitive and specific than creatinine, suggesting that despite the normal creatinine values, kidney impairment persisted, such that these miRNAs had a high prognosis value with respect to the progression of these patients over time into long-term chronic renal impairment.

**Table 2: ROC curve analysis for miRNAs in ARF patients in ICU on day 7.**

| Contrast result variables | Area | Standard Error ^{a} | Asymptotic Sig. ^{b} | 95% Asymptotic Confidence Interval | |
|---|---|---|---|---|---|
| | | | | Lower limit | Upper limit |
| miR_101_D7 | 0.886 | 0.107 | 0.003 | 0.676 | 1.096 |
| miR_210_D7 | 0.687 | 0.133 | 0.407 | 0.346 | 0.869 |
| miR_126_D7 | 0.729 | 0.106 | 0.077 | 0.520 | 0.937 |
| miR_26b_D7 | 0.986 | 0.019 | 0.000 | 0.949 | 1.022 |
| miR_29a_D7 | 0.625 | 0.137 | 0.333 | 0.357 | 0.893 |
| miR_146a_D7 | 1.000 | 0.000 | 0.000 | 1.000 | 1.000 |
| miR_27a_D7 | 0.571 | 0.118 | 0.580 | 0.339 | 0.804 |
| miR_93_D7 | 0.364 | 0.136 | 0.293 | 0.094 | 0.634 |
| miR_10a_D7 | 1.000 | 0.000 | 0.000 | 1.000 | 1.000 |
| miR_127_D7 | 0.829 | 0.104 | 0.319 | 0.424 | 0.833 |

Data of Table 3 shows that the alteration in these miRNAs indicated a predisposition to developing ischemic ARF after cardiac surgery.

**Table 3: ROC curve analysis for miRNAs in patients after cardiac surgery.**

| Contrast result variables | Area | Standard Error ^{a} | Asymptotic Sig. ^{b} | 95% Asymptotic Confidence Interval | |
|---|---|---|---|---|---|
| | | | | Lower limit | Upper limit |
| miR101_b | 0.899 | 0.065 | 0.001 | 0.772 | 1.026 |
| miR210_b | 0.702 | 0.112 | 0.082 | 0.482 | 0.911 |
| miR126_b | 0.848 | 0.075 | 0.003 | 0.701 | 0.996 |
| miR26b_b | 0.944 | 0.039 | 0.000 | 0.869 | 1.020 |
| miR29a_b | 0.803 | 0.089 | 0.009 | 0.629 | 0.977 |
| miR146a_b | 0.778 | 0.087 | 0.017 | 0.608 | 0.947 |
| miR27a_b | 0.896 | 0.064 | 0.001 | 0.771 | 1.021 |
| miR93_b | 0.934 | 0.049 | 0.000 | 0.838 | 1.301 |
| miR10a_b | 0.697 | 0.127 | 0.090 | 0.448 | 0.946 |
| miR127_b | 0.859 | 0.068 | 0.002 | 0.726 | 0.991 |

**Table 4: ROC curve analysis for miRNAs in patients immediately after cardiac surgery.**

| Data of Table 4 shows that the alteration in these miRNAs are early indicators of ARF development after cardiac surgery. | | | | | |
|---|---|---|---|---|---|
| Contrast result variables | Area | Standard Error ^{a} | Asymptotic Sig. ^{b} | 95% Asymptotic Confidence Interval | |
| | | | | Lower limit | Upper limit |
| miR101_pi | 0.667 | 0.118 | 0.154 | 0.434 | 0.899 |
| miR210_pi | 0.598 | 0.119 | 0.402 | 0.365 | 0.831 |
| miR126_pi | 0.667 | 0.119 | 0.154 | 0.434 | 0.899 |
| miR26b_pi | 0.757 | 0.101 | 0.028 | 0.558 | 1.955 |
| miR29a_pi | 0.561 | 0.110 | 0.603 | 0.344 | 0.777 |
| miR146a_pi | 0.534 | 0.120 | 0.769 | 0.299 | 0.770 |
| miR27a_pi | 0.831 | 0.091 | 0.005 | 0.652 | 1.009 |
| miR93_pi | 0.852 | 0.078 | 0.003 | 0.699 | 1.004 |
| miR10a_pi | 0.836 | 0.098 | 0.004 | 0.644 | 1.028 |
| miR127_pi | 0.751 | 0.092 | 0.032 | 0.570 | 0.932 |

## Claims

1. A method for the diagnosis of acute renal damage as a result of ischemic or toxic etiology which comprises determining the expression level of at least miR-26b in a serum or blood sample isolated from a subject and comparing said expression level with a control value, wherein the reduction of said expression level is indicative of acute renal damage as a result of ischemic or toxic etiology.

2. The method for the diagnosis of acute renal damage according to claim 1, where the expression of the micro-RNA is determined by means of quantitative PCR.

3. The method according to any of the preceding claims, where the expression level of the micro-RNA is determined by means of RNA microarrays.

4. Method according to any of the preceding claims, **characterized in that** it further comprises determining the expression levels of miR-29a, miR-454, miR-146a, miR-27a, mi-R93 and miR-10a collectively.

5. Method according to claim 4, where the reduction in the expression level of at least one of the micro-RNAs is indicative of acute renal damage.

6. *In vitro* use of at least miR-26b for the diagnosis of acute renal damage as a result of ischemic or toxic etiology in a serum or blood sample isolated from a subject.

7. *In vitro* use according to claim 6 which further comprises determining the expression level of miR-29a, miR-454, miR-146a, miR-27a, mi-R93 and miR-10a, collectively, for the diagnosis and prognosis of acute renal damage.

8. *In vitro* use of a kit for the diagnosis of acute renal damage as a result of ischemic or toxic etiology according to the method of claims 1-5, comprising the probes and primers required for determining the expression level of at least miR-26b.

## Patentansprüche

1. Verfahren zur Diagnose von akuter Nierenschädigung als Ergebnis ischämischer oder toxischer Ätiologie, welches das Bestimmen des Expressionsniveaus mindestens von miR-26b in einer Serum- oder Blutprobe, welche aus einer Testperson isoliert wurde, und das Vergleichen des genannten Expressionsniveaus mit einem Steuerwert umfasst, wobei die Verringerung des genannten Expressionsniveaus auf eine akute Nierenschädigung als Ergebnis ischämischer oder toxischer Ätiologie hindeutet.

2. Verfahren zur Diagnose von akuter Nierenschädigung nach Anspruch 1, wobei die Expression der Mikro-RNS mittels quantitativer PCR bestimmt wird.

3. Verfahren nach einem der vorhergehenden Ansprüche, wobei das Expressionsniveau der Mikro-RNS mittels RNS-Mikroarrays bestimmt wird.

4. Verfahren nach einem der vorhergehenden Ansprüche, **dadurch gekennzeichnet, dass** es zusätzlich das Bestimmen der Expressionsniveaus von miR-29a, miR-454, miR-146a, miR-27a, mi-R93 und miR-10a gemeinsam umfasst.

5. Verfahren nach Anspruch 4, wobei die Verringerung beim Expressionsniveau mindestens von einer der Mikro-RNS auf eine akute Nierenschädigung hindeutet.

6. *In vitro*-Verwendung mindestens von miR-26b zur Diagnose von akuter Nierenschädigung als Ergebnis ischämischer oder toxischer Ätiologie in einer Serum- oder Blutprobe, welche aus einer Testperson isoliert wurde.

7. *In vitro*-Verwendung nach Anspruch 6, welche zusätzlich das Bestimmen des Expressionsniveaus von miR-29a, miR-454, miR-146a, miR-27a, mi-R93 und miR-10a gemeinsam umfasst, zur Diagnose und Prognose von akuter Nierenschädigung.

8. *In vitro*-Verwendung eines Kits zur Diagnose von akuter Nierenschädigung als Ergebnis ischämischer oder toxischer Ätiologie gemäß dem Verfahren nach den Ansprüchen 1-5, umfassend die Sonden und Primer, welche zur Bestimmung des Expressionsniveaus mindestens von miR-26b benötigt werden.

## Revendications

1. Procédé de diagnostic de l'insuffisance rénale aiguë résultant d'une étiologie ischémique ou toxique qui comprend la détermination du niveau d'expression d'au moins miR-26b dans un échantillon de sérum ou de sang isolé d'un sujet et la comparaison dudit niveau d'expression avec une valeur de contrôle, dans lequel la réduction dudit niveau d'expression est révélatrice de l'insuffisance rénale aiguë résultant d'une étiologie ischémique ou toxique.

2. - Procédé de diagnostic de l'insuffisance rénale aiguë selon la revendication 1, où l'expression du micro-ARN est déterminée par le biais de PCR quantitative.

3. - Procédé selon l'une quelconque des revendications précédentes, où le niveau d'expression du micro-ARN est déterminé par le biais de puces à ARN.

4. - Procédé selon l'une quelconque des revendications précédentes, **caractérisé en ce qu'**il comprend en outre la détermination des niveaux d'expression de miR-29a, miR-454, miR-146a, miR-27a, mi-R93 et miR-10a collectivement.

5. - Procédé selon la revendication 4, où la réduction dans le niveau d'expression d'au moins un des micro-ARN est révélatrice de l'insuffisance rénale aigüe.

6. - Utilisation *in vitro* d'au moins miR-26b pour le diagnostic de l'insuffisance rénale aigüe résultant d'une étiologie ischémique ou toxique dans un échantillon de sérum ou de sang isolé d'un sujet.

7. - Utilisation *in vitro* selon la revendication 6 qui comprend en outre la détermination du niveau d'expression de miR-29a, miR-454, miR-146a, miR-27a, mi-R93 et miR-10a, collectivement, pour le diagnostic et le pronostic de l'insuffisance rénale aigüe.

8. - Utilisation *in vitro* d'une trousse de diagnostic de l'insuffisance rénale aigüe résultant d'une étiologie ischémique ou toxique selon le procédé des revendications 1-5, comprenant les sondes et les amorces nécessaires pour la détermination du niveau d'expression d'au moins miR-26b.
